(19) Europäisches Patentamt
European Patent Office
Office européen des brevets

(11) Veröffentlichungsnummer: **0 019 182**
**B1**

(12) # EUROPÄISCHE PATENTSCHRIFT

(45) Veröffentlichungstag der Patentschrift: **07.07.82**

(21) Anmeldenummer: **80102430.8**

(22) Anmeldetag: **05.05.80**

(51) Int. Cl.³: **C 07 D 413/10,** C 07 D 413/14, D 06 L 3/12 // (C07D413/10, 307/00, 263/00),(C07D413/14, 307/00, 263/00, 231/00, 249/00)

(54) **Benzofuranverbindungen sowie deren Verwendung als optische Aufheller.**

(30) Priorität: **11.05.79 DE 2918965**

(43) Veröffentlichungstag der Anmeldung: **26.11.80 Patentblatt 80/24**

(45) Bekanntmachung des Hinweises auf die Patenterteilung: **07.07.82 Patentblatt 82/27**

(84) Benannte Vertragsstaaten: **CH DE FR GB IT LI**

(56) Entgegenhaltungen:
**CH-A-561 719**
**CH-B-512 623**
**DE-A-2 031 774**
**DE-A-2 162 439**
**DE-A-2 238 734**
**DE-A-2 400 656**
**DE-A1-2 724 368**
**GB-A-1 314 368**
**US-A-3 796 707**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Wehling, Bernhard, Dr., Andreas-Gryphius-Strasse 26, D-5000 Köln 80 (DE)**

**EP 0 019 182 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Benzofuranverbindungen sowie deren Verwendung als optische Aufheller

Die Erfindung betrifft Verbindungen der Formel

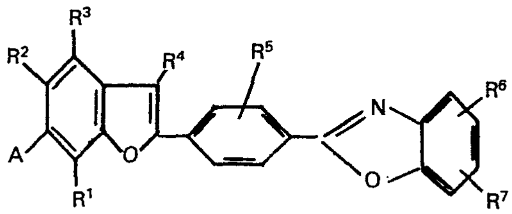

(I)

in der

$R^1$, $R^2$, $R^3$ Wasserstoff, Methyl, Ethyl, Methoxy, Chlor,

durch $C_1$–$C_4$-Alkyl und/oder $C_1$–$C_4$-Alkoxy substituiertes Phenyl oder ein Substituent der allgemeinen Formel

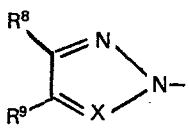

in der

$R^8$ $C_1$–$C_4$-Alkyl, $C_1$–$C_4$-Alkoxy, ein Phenylrest, der gegebenenfalls durch $C_1$–$C_4$-Alkyl, Phenyl, $C_1$–$C_4$-Alkoxy oder Chlor substituiert ist, Benzyl oder Styryl,

$R^9$ Wasserstoff, Cyan, Carboxy, $C_1$–$C_4$-Alkylcarbonylamino, Benzoylamino oder $R^8$,

$R^8$ und $R^9$ zusammen einen ankondensierten Naphthalinring oder Benzolring, der gegebenenfalls durch $C_1$–$C_4$-Alkyl und/oder $C_1$–$C_4$-Alkoxy substituiert sein kann,

X N oder $C(R^{10})$,

$R^{10}$ Wasserstoff, $C_1$–$C_4$-Alkyl, Phenyl,

$R^4$ Wasserstoff, Alkyl mit 1 bis 4 C-Atomen, Vorzugsweise Methyl oder gegebenenfalls durch Methyl und/oder Methoxy substituiertes Phenyl,

$R^5$ Wasserstoff, Chlor, $C_1$–$C_4$-Alkyl,

$R^6$ Wasserstoff, Alkyl mit 1 bis 4 C-Atomen, vorzugsweise Methyl, Methoxy, Chlor, Alkylsulfonyl mit 1 bis 4 C-Atomen, Cyan, COOH, $SO_3H$, $COOR^{11}$, $SO_3R^{11}$ oder $CON(R^4)_2$,

$R^7$ Wasserstoff, $C_1$–$C_4$-Alkyl, Methoxy oder Chlor,

$R^{11}$ $C_{1-4}$-Alkyl, vorzugsweise Methyl, $C_2$–$C_4$-Hydroxyalkyl, Cyanethyl, gegebenenfalls durch Chlor, Methyl oder Methoxy substituiertes Phenyl, Cyclohexyl oder Benzyl

bedeuten.

Die Herstellung der Verbindungen der Formel (I) kann nach verschiedenen Methoden erfolgen. Eine Variante ist dadurch gekennzeichnet, dass man in an sich bekannter Weise (vgl. DE-OS 2 162 439) Verbindungen der formel

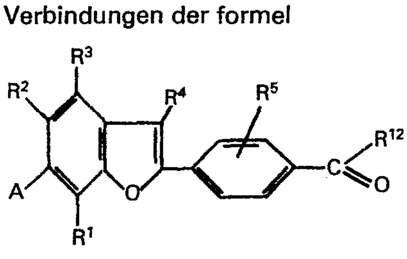

(II)

in der

$R^{12}$ Alkoxy, Chlor oder Brom bedeutet, mit Verbindungen der Formel

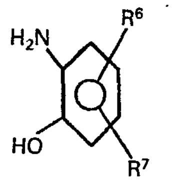

(III)

umsetzt.

Weiterhin können die Verbindungen der Formel (I) dadurch hergestellt werden, dass man Verbindungen der Formel

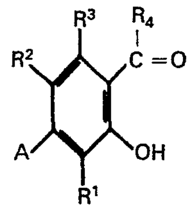

(IV)

bzw. deren Salze mit Verbindungen der Formel

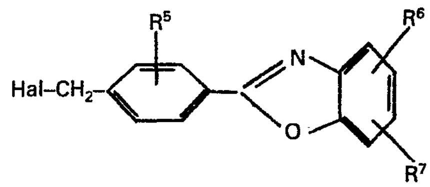

(V)

in der Hal Chlor oder Brom bedeutet, zunächst zu Produktion der Formel

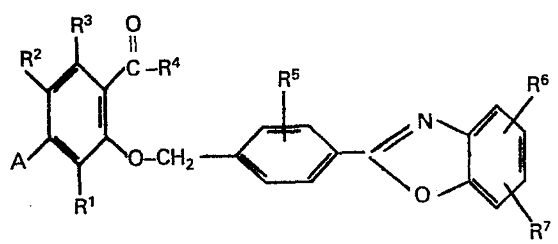

(VI)

umsetzt und diese dann zu Verbindungen der Formel (I) cyclisiert.

Durch Umsetzung einer Verbindung der Formel

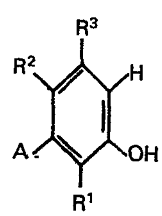

(VII)

mit einer Verbindung der Formel

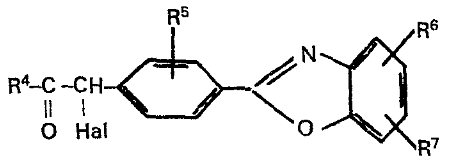

(VIII)

lassen sich ebenfalls Verbindungen der Formel (I) herstelllen.

Auch durch Umsetzung von Verbindungen der Formel (VII) mit Verbindungen der Formel

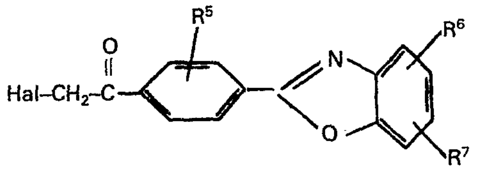

(IX)

können über die Zwischenprodukte der Formel

(X)

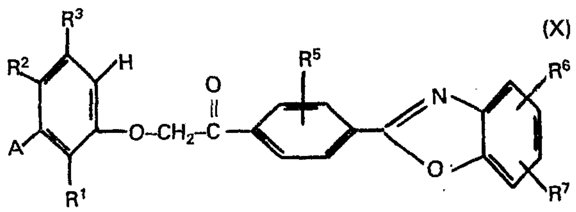

durch Kondensation und anschliessende Umlagerung Verbindungen der Formel (I) mit $R^4 = H$ gebildet werden.

Die neuen Verbindungen eignen sich als optische Aufheller für natürliche und synthetische Fasern, insbesondere Polyester, Polyamide, Celluloseester und Polyacrylnitril. Sie sind ebenso zur Masseaufhellung dieser Substrate geeignet.

Gegenüber einem nächstvergleichbaren bekannten Benzofuranaufheller gemäss DE-A 2 162 439 zeichnen sich die neuen Verbindungen durch eine bessere Lichtechtheit ihrer Polyesterfärbungen aus.

Beispiel 1

5,9 g 2-Aminophenol werden in 120 ml Pyridin gelöst und 20 g 2-(4-Chlorcarbonyl-phenyl)-6-(4-methyl-5-phenyl-1,2,3-triazol-2-yl)-benzofuran innerhalb von 15 Minuten in kleinen Portionen zugegeben. Man erwärmt 8 Stunden auf 80 °C. Anschliessend wird das Reaktionsgemisch auf Raumtemperatur abgekühlt, auf 1000 ml 10%ige Salzsäure gegossen, abgesaugt, neutral gewaschen und getrocknet. Das Zwischenprodukt der Formel

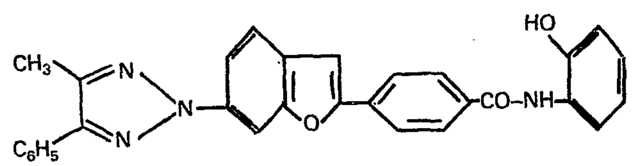

(101)

wird in einem Gemisch aus 140 ml Trichlorbenzol und 0,2 g Borsäure 4 Stunden auf 200 °C erhitzt und das entstehende Wasser abdestilliert. Dann wird kaltgerührt, abgesaugt, mit Methanol gewaschen und bei 100 °C getrocknet. Nach dem Umkristallisieren aus Glykolmonomethylether/A-Kohle verbleiben 13,5 g (60% der Theorie) der Verbindung der Formel

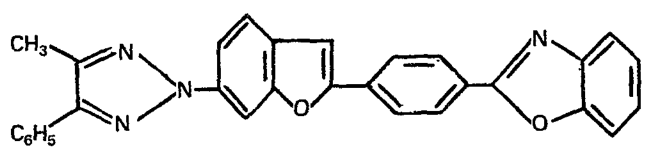

(102)

die in DMF gelöst blau fluoresziert.

Das 2-(4-Chlorcarbonyl-phenyl)-6-(4-methyl-5-phenyl-1,2,3-triazol-2-yl)-benzofuran wird folgendermassen hergestellt:

100 g 4-(4-Methyl-5-phenyl-1,2,3-traiazol-2-yl)-salicyl-aldehyd, 47 g 4-Cyanobenzylchlorid und 62,2 g Kaliumcarbonat werden in 700 ml Dimethylsulfoxid 8 Stunden auf 80 °C erhitzt. Das Reaktionsgemisch wird anschliessend auf 3000 ml Wasser gegossen, mit konzentrierter Salzsäure kongosauer gestellt, abgesaugt und neutral gewaschen. Das noch feuchte Zwischenprodukt wird in 1300 ml Dimethylformamid bei 80 °C gelöst, bei 30 °C werden 76 g Kaliumhydroxid-Pulver (88%-ig) zugesetzt und 5 Stunden bei 30 °C gerührt. Man giesst auf 5000 ml Wasser, stellt mit konzentrierter HCl kongosauer, saugt ab, wäscht neutral und trocknet bei 100 °C. Das Rohprodukt wird in 1200 ml Glykolmonomethylether und 730 ml 50%iger Kalilauge 13 Stunden refluxiert. Man giesst auf ein Gemisch von 970 ml konzentrierter Salzsäure und 1940 g Eis, saugt den Niederschlag ab, wäscht neutral und trocknet bei 100 °C. Ausbeute: 95 g (80,2% der Theorie) (2-(4-Carboxyphenyl)-6-(4-methyl-5-phenyl-1,2,3-triazol-2-yl)-benzofuran vom Fp. 275 °C. Die Carbonsäure wird in 940 ml destilliertem Toluol verrührt, mit 117,5 g Thionylchlorid und 5 ml Dimethylformamid versetzt und allmählich zum Sieden erhitzt. Man refluxiert 1 Stunde, klärt mit A-Kohle, kühlt das Filtrat ab, saugt ab und trocknet im Vakuum bei 80 °C.

Ausbeute: 71,6 g (72% der Theorie) 2-(4-Chlorcarbonylphenyl)-6-(4-methyl-5-phenyl-1,2,3-triazol-2-yl)-benzofuran vom Fp. 201 °C.

Beispiel 2

Die Verbindung der Formel (101) kann auch auf folgendem Weg hergestellt werden.

9,2 g 4-(4-Methyl-5-phenyl-1,2,3-triazol-2-yl)-salicylaldehyd, Natriumsalz (98,2%ig) und 7,3 g 2-(4-Chlormethyl-phenyl)-benzoxazol werden in 250 ml Dimethylformamid 8 Stunden bei 70 °C gerührt. Anschliessend gibt man bei Raumtemperatur 14,2 g Kalium-tert.-butylat (95%ig) zu und rührt 4 Stunden bei 60 °C. Nach dem Ausgiessen auf 500 ml Wasser wird abgesaugt, neutral gewaschen und bei 100 °C getrocknet. Aus Glykolmonomethylether/A-Kohle umkristallisiert erhält man 12,2 g (86,9% der Theorie) einer Verbindung, die mit der Verbindung der Formel (101) identisch ist und in Dimethylformamidlösung blau fluoresziert.

Beispiel 3

Ersetzt man das 2-(4-Chlormethyl-phenyl)-benzoxazol durch die entsprechend substituierten Analoga und verfährt im übrigen wie in Beispiel 2 beschrieben so erhält man folgende Verbindungen der Formel

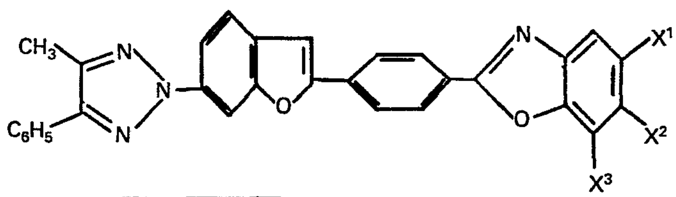

| Nr. | $X^1$ | $X^2$ | $X^3$ | Fluoreszenzfarbe in DMF-Lösung |
|---|---|---|---|---|
| 301 | $CH_3$ | H | H | blau |
| 302 | $CH_3$ | H | $CH_3$ | blau |
| 303 | $CH_3$ | $CH_3$ | H | blau |
| 304 | $C(CH_3)_3$ | H | H | blau |
| 305 | Cl | H | H | blaugrün |
| 306 | $CO_2CH_3$ | H | H | blaugrün |

Beispiel 4

Wenn man das Natriumsalz des 4-(4-Methyl-5-phenyl-1,2,3-triazol-2-yl)-salicylaldehyds durch das Natriumsalz des 4-(4-Ethyl-5-methyl-1,2,3-triazol-2-yl)-salicylaldehyds ersetzt, so erhält man in Analogie zu Beispiel 2 die Verbindungen der Formel:

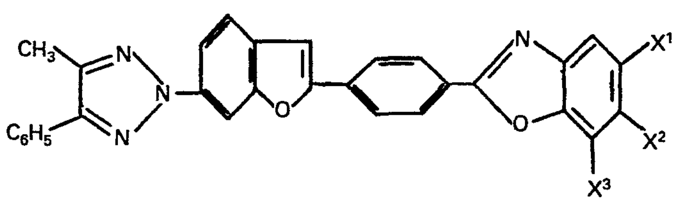

| Nr. | $X^1$ | $X^2$ | $X^3$ | Fluoreszenzfarbe in DMF-Lösung |
|---|---|---|---|---|
| 401 | H | H | H | blauviolett |
| 402 | $CH_3$ | H | H | blau |
| 403 | $CH_3$ | H | $CH_3$ | blau |
| 404 | $CH_3$ | $CH_3$ | H | blau |
| 405 | $C(CH_3)_3$ | H | H | blau |
| 406 | Cl | H | H | blaugrün |
| 407 | $CO_2CH_3$ | H | H | blaugrün |

Beispiel 5

Unter Verwendung des Natriumsalzes des 4-(Naphth/1,2-d/1,2,3-triazol-2-yl)-salicylaldehyds erhält man in Analogie zu Beispiel 2 die Verbindungen der Formel:

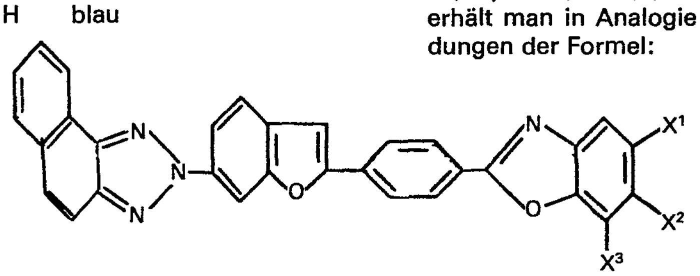

| Nr. | $X^1$ | $X^2$ | $X^3$ | Fluoreszenzfarbe in DMF-Lösung |
|---|---|---|---|---|
| 501 | H | H | H | blau |
| 502 | $CH_3$ | H | H | blaugrün |
| 503 | $CH_3$ | H | $CH_3$ | blaugrün |
| 504 | $CH_3$ | $CH_3$ | H | blaugrün |
| 505 | $C(CH_3)_3$ | H | H | blaugrün |
| 506 | Cl | H | H | blaugrün |
| 507 | $CO_2CH_3$ | H | H | blaugrün |

Beispiel 6

Unter Einsatz des Natriumsalzes des 4-Phenylsalicylaldehyds bzw. des 2-Hydroxy-4-phenyl-ace-

tophenons erhält man in Analogie zu Beispiel 2 die Verbindungen der Formel:

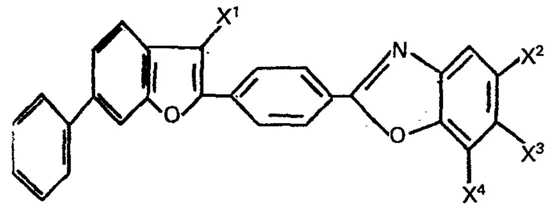

| Nr. | $X^1$ | $X^2$ | $X^3$ | $X^4$ | Fluoreszenzfarbe in DMF-Lösung |
|---|---|---|---|---|---|
| 601 | H | H | H | H | violett |
| 602 | H | $CH_3$ | H | H | blauviolett |
| 603 | H | $CH_3$ | H | $CH_3$ | blau |
| 604 | H | $CH_3$ | $CH_3$ | H | blau |
| 605 | H | $C(CH_3)_3$ | H | H | blauviolett |
| 606 | H | Cl | H | H | blau |
| 607 | H | $CO_2CH_3$ | H | H | blaugrün |
| 608 | $CH_3$ | H | H | H | violett |
| 609 | $CH_3$ | $CH_3$ | H | H | blauviolett |
| 610 | $CH_3$ | $CH_3$ | H | $CH_3$ | blau |
| 611 | $CH_3$ | $CH_3$ | $CH_3$ | H | blau |
| 612 | $CH_3$ | $C(CH_3)_3$ | H | H | blauviolett |
| 613 | $CH_3$ | Cl | H | H | blauviolett |
| 614 | $CH_3$ | $CO_2CH_3$ | H | H | blau |

**Patentansprüche**

1. Benzofuranverbindungen der Formel

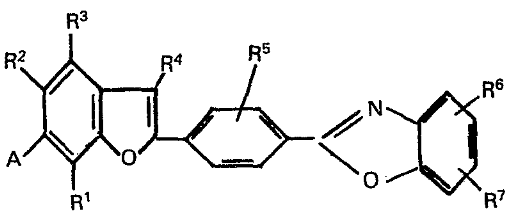

(I)

in der

$R^1$, $R^2$, $R^3$ Wasserstoff, Methyl, Ethyl, Methoxy, Chlor,

A durch $C_1$–$C_4$-Alkyl und/oder $C_1$–$C_4$-Alkoxy substituiertes Phenyl oder ein Substituent der allgemeinen Formel

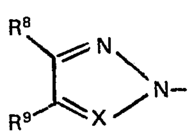

in der

$R^8$ $C_1$–$C_4$-Alkyl, $C_1$–$C_4$-Alkoxy, ein Phenylrest, der gegebenenfalls durch $C_1$–$C_4$-Alkyl, Phenyl, $C_1$–$C_4$-Alkoxy oder Chlor substituiert ist, Benzyl oder Styryl,

$R^9$ Wasserstoff, Cyan, Carboxy, $C_1$–$C_4$-Alkylcarbonylamino, Benzoylamino oder $R^8$,

$R^8$ und $R^9$ zusammen einen ankondensierten Naphthalinring oder Benzolring, der gegebenenfalls durch $C_1$–$C_4$-Alkyl und/oder $C_1$–$C_4$-Alkoxy substituiert sein kann,

X N oder $C(R^{10})$

$R^{10}$ Wasserstoff, $C_1$–$C_4$-Alkyl, Phenyl,

$R^4$ Wasserstoff, Alkyl mit 1 bis 4 C-Atomen, vorzugsweise Methyl oder gegebenenfalls durch Methyl und/oder Methoxy substituiertes Phenyl,

$R^5$ Wasserstoff, Chlor, $C_1$–$C_4$-Alkyl,

$R^6$ Wasserstoff, Alkyl mit 1 bis 4 C-Atomen, vorzugsweise Methyl, Methoxy, Chlor, Alkylsulfonyl mit 1–4 C-Atomen, Cyan, COOH, $SO_3H$, $COOR^{11}$, $SO_3R^{11}$ oder $CON(R^4)_2$,

$R^7$ Wasserstoff, $C_1$–$C_4$-Alkyl, Methoxy oder Chlor,

$R^{11}$ $C_1$–$C_4$-Alkyl, vorzugsweise Methyl, $C_2$–$C_4$-Hydroxyalkyl, Cyanethyl, gegebenenfalls durch Chlor, Methyl oder Methoxy substituiertes Phenyl, Cyclohexyl oder Benzyl

sein können.

2. Benzofuranverbindungen gemäss Anspruch 1, dadurch gekennzeichnet, dass A für

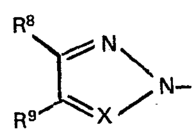

steht.

3. Benzofuranverbindungen gemäss Anspruch 1, dadurch gekennzeichnet, dass A für gegebenenfalls durch $C_1$–$C_4$-Alkyl und/oder $C_1$–$C_4$-Alkoxy substituiertes Phenyl steht.

4. Benzofuranverbindung gemäss Anspruch 1 der Formel

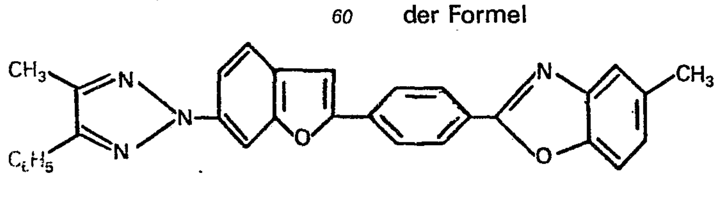

5. Benzofuranverbindung gemäss Anspruch 1 der Formel

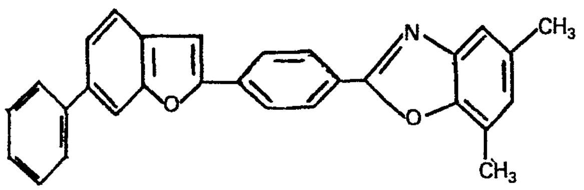

6. Verwendung der Benzofuranverbundungen gemäss Ansprüchen 1–5 als optische Aufheller.

**Revendications**

1. Composés du benzofuranne de formule:

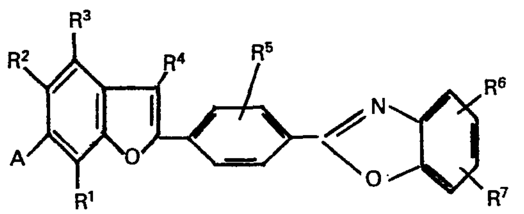

(I)

dans laquelle

$R^1$, $R^2$, $R^3$ représentent de l'hydrogène, un groupe méthyle, un groupe éthyle, un groupe méthoxy, du chlore,

A est un groupe phényle substitué par un radical alkyle en $C_1$ à $C_4$ et/ou alkoxy en $C_1$ à $C_4$ ou un substituant de formule générale

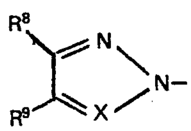

dans laquelle

$R^8$ est un reste alkyle en $C_1$ à $C_4$, un reste alkoxy en $C_1$ à $C_4$, un reste phényle qui est éventuellement substitué par un radical alkyle en $C_1$ à $C_4$, phényle, alkoxy en $C_1$ à $C_4$ ou du chlore, un reste benzyle ou un reste styryle,

$R^9$ est l'hydrogène, le groupe cyano, le groupe carboxy, un groupe (alkyle en $C_1$ à $C_4$)-carbonylamino, benzoylamino ou $R^8$,

$R^8$ et $R^9$ forment ensemble un noyau de naphtalène ou un noyau de benzène condensé qui peut éventuellement être substitué par un reste alkyle en $C_1$ à $C_4$ et/ou alkoxy en $C_1$ à $C_4$,

X représente N ou $C(R^{10})$

$R^{10}$ est l'hydrogène, un reste alkyle en $C_1$ à $C_4$ ou le reste phényle,

$R^4$ est l'hydrogène, un reste alkyle ayant 1 à 4 atomes de carbone, de préférence méthyle ou un reste phényle éventuellement substitué par un radical méthyle et/ou méthoxy,

$R^5$ est l'hydrogène, le chlore, un reste alkyle en $C_1$ à $C_4$,

$R^6$ est l'hydrogène, un reste alkyle ayant 1 à 4 atomes de carbone, de préférence le reste méthyle, un groupe méthoxy, du chlore, un reste alkylsulfonyle ayant 1 à 4 atomes de carbone, cyano, COOH, $SO_3H$, $COOR^{11}$, $SO_3R^{11}$ ou $CON(R^4)_2$,

$R^7$ est l'hydrogène, un reste alkyle $C_1$ à $C_4$, le reste méthoxy ou du chlore,

$R^{11}$ est un reste alkyle en $C_1$ à $C_4$, de préférence méthyle, un reste hydroxyalkyle en $C_2$ à $C_4$, cyanéthyle, phényle éventuellement substitué par du chlore, un radical méthyle ou méthoxy, cyclohexyle ou benzyle.

2. Composés du benzofuranne suivant la revendication 1, caractérisés en ce que A est le reste

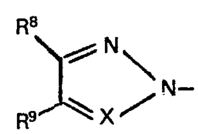

3. Composés du benzofuranne suivant la revendication 1, caractérisés en ce que A est un reste phényle éventuellement substitué par un radical alkyle en $C_1$ à $C_4$ et/ou alkoxy en $C_1$ à $C_4$.

4. Composé du benzofuranne suivant la revendication 1, de formule:

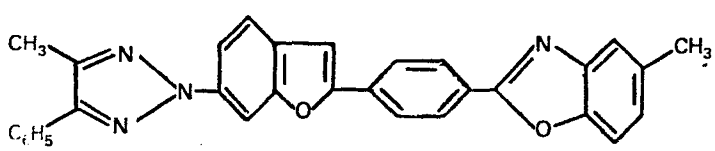

5. Composé du benzofuranne suivant la revendication 1, de formule:

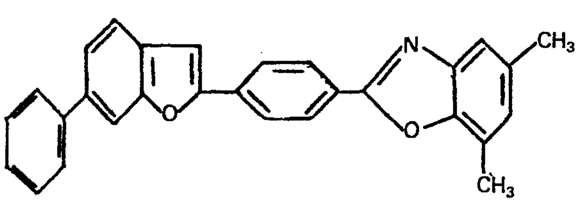

6. Utilisation des composés du benzofuranne suivant les revendications 1 à 5 comme azurants optiques.

**Claims**

1. Benzofurane compounds of the formula

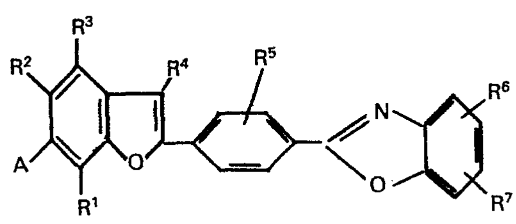

(I)

in which

$R^1$, $R^2$ and $R^3$ can be hydrogen, methyl, ethyl, methoxy or chlorine,

A can be phenyl which is substituted by $C_1$–$C_4$-alkyl and/or $C_1$–$C_4$-alkoxy, or a substituent of the general formula

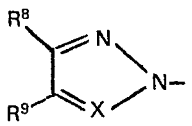

in which

$R^8$ can be $C_1$–$C_4$-alkyl, $C_1$–$C_4$-alkoxy, a phenyl radical which is optionally substituted by $C_1$–$C_4$-alkyl, phenyl, $C_1$–$C_4$-alkoxy or chlorine, benzyl or styryl and

$R^9$ can be hydrogen, cyano, carboxyl, $C_1$–$C_4$-alkyl-carbonylamino, benzoylamino or $R^8$, or

$R^8$ and $R^9$ together can be a fused-on naphthalene or benzene ring, which can optionally be substituted by $C_1$–$C_4$-alkyl and/or $C_1$–$C_4$-alkoxy, and X can be N or $C(R^{10})$,

$R^{10}$ can be hydrogen, $C_1$–$C_4$-alkyl or phenyl,

$R^4$ can be hydrogen, alkyl with 1 to 4 C atoms, preferably methyl, or phenyl which is optionally substituted by methyl and/or methoxy,

$R^5$ can be hydrogen, chlorine or $C_1$–$C_4$-alkyl,

$R^6$ can be hydrogen, alkyl with 1 to 4 C atoms, preferably methyl, methoxy, chlorine, alkyl-sulphonyl with 1 to 4 C atoms, cyano, COOH, $SO_3H$, $COOR^{11}$, $SO_3R^{11}$ or $CON(R^4)_2$,

$R^7$ can be hydrogen, $C_1$–$C_4$-alkyl, methoxy or chlorine and

$R^{11}$ can be $C_1$–$C_4$-alkyl, preferably methyl, $C_2$–$C_4$-hydroxyalkyl, cyanoethyl, phenyl which is optionally substituted by chlorine, methyl or methoxy, cyclohexyl or benzyl.

2. Benzofurane compounds according to Claim 1, characterised in that A represents

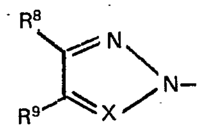

3. Benzofurane compounds according to Claim 1, characterised in that A represents pehnyl which is optionally substituted by $C_1$–$C_4$-alkyl and/or $C_1$–$C_4$-alkoxy.

4. Benzofurane compound according to Claim 1, of the formula

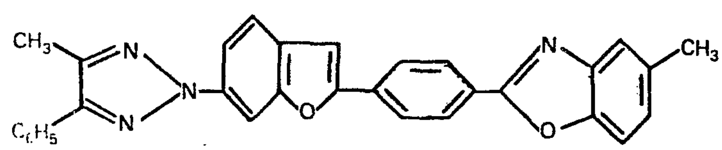

5. Benzofurane compound according to Claim 1, of the formula

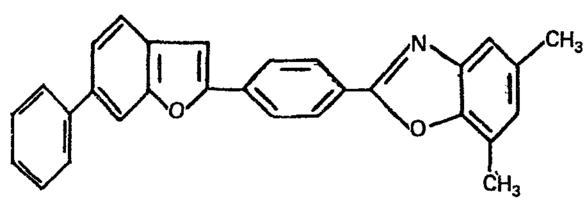

6. Use of the benzofurane compounds accord-ing to Claims 1–5 as optical brighteners.